(19)

Europäisches
Patentamt
European
Patent Office
Office européen
des brevets

(11) **EP 1 912 068 A1**

(12) **EUROPEAN PATENT APPLICATION**

(43) Date of publication:
**16.04.2008 Bulletin 2008/16**

(51) Int Cl.:
***G01N 33/543*** (2006.01)   ***C12Q 1/68*** (2006.01)
***G01N 33/68*** (2006.01)

(21) Application number: **07118308.1**

(22) Date of filing: **11.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **12.10.2006 EP 06122172**

(71) Applicant: **Eppendorf Array Technologies SA 5000 Namur (BE)**

(72) Inventors:
• **Remacle, José**
  **5020 Malonne (BE)**
• **Mainfroid, Véronique**
  **4300 Waremme (BE)**
• **Margaine, Sylvain**
  **1070 Anderlecht (BE)**

(74) Representative: **pronovem**
  **Office Van Malderen**
  **Avenue Josse Goffin 158**
  **1082 Bruxelles (BE)**

(54) **Method and system for quantification of a target compound obtained from a biological sample upon chips**

(57)    The present invention is related to a method for a quantification of a target compound (1) (being a polynucleotide or a protein) present in a sample solution, comprising the steps of:

a) Possibly diluting the target compound (1) by a known dilution factor,

b) possibly labeling the target compound (1),

c) putting into contact the target compound (1) with a capture probe (2), in order to allow a specific binding between the said target compound (1) and the said capture probe (2), the said capture probe (2) being fixed upon a surface of a solid support (3) according to an array comprising a density of at least 10 discrete regions per cm², each of said discrete regions (A) being fixed with a species of capture probe (2) capable of specifically binding to a target compound (1), wherein at least 4 additional discrete regions (B) of the solid support (3) surface are printed with solutions containing known different and increased concentrations of a (labeled) detection molecule (4, 4', 4", 4'''), said detection molecule being from the same family, either a polynucleotide or a protein, as the capture probes (2),wherein each of said discrete regions (A,B) have a diameter comprised between 50 and 500 μm,

d) detecting by the same detection means signals resulting from the binding between the target compound (1) and its corresponding capture probe (2) and resulting from the printed detection molecule (4, 4', 4", 4''') in the different discrete regions (A, B),

e) obtaining a detection curve (C) of the detected signals of said detection molecule (4, 4', 4", 4''') in the at least 4 additional discrete regions (B) of the solid support (3) surface as a function of their corresponding printed detection solution concentrations in arbitrary units (AU),

f) converting the signal obtained from the target compound (1) bound to a specific capture probe (2) into a concentration value presented in arbitrary units (AU) by a conversion step upon the detection curve (C) of the detected signal, and

g) quantifying the target compound by converting said concentration value into a target amount using a target concentration curve.

FIG. 10

**Description**

**Field of the invention**

The present invention is related to a method and system for a quantification of a target compound obtained from a biological sample after its binding to a capture probe fixed upon chips.

The present invention is also related to a method and system for obtaining a concentration curve of a target compound by combining results obtained on different arrays and that includes a quantification software that allows calculating the concentration of target compounds in a biological sample.

**Background on the invention and state of the art**

Biological assays are based upon interaction specificity between two biological molecules such as two strands of nucleic acid molecules, an antigen with a corresponding antibody or a ligand and its receptor. The present challenge of biological assays is to perform simultaneously detection of multiple target molecules present in a sample. Miniaturized assays developed upon the surface of "biochips" are tools that allow multiplex reactions in a microscopic format, said detection being made with a limited volume of sample for the screening and/or the identification of multiple possible target compounds. These arrays are formed of discrete regions or specific locations, each containing (a) specific capture probe(s) used for the binding of the target compound(s). These discrete regions or locations as small as a few micrometers, allow the fixation of hundreds and even thousands capture probes per cm$^2$ surface (WO 95/11995).

Once bound to their capture probes, target compounds have to be labeled in order to be detected. The methods for their detection are limited given the very low amounts of material, often in the femtomoles or even attomoles range, that are present in one location. Two labeling methods are mainly used. The first one is fluorescence. Labeling of a target compound being DNA or (m) RNA is performed either by direct incorporation of labeled nucleotides during the copy or duplication of the DNA or RNA. However, since the incorporation of the labeled nucleotides is not very efficient, indirect labeling is also proposed that uses a first non fluorescent label such as biotin or a hapten and then a second fluorescent label such as fluorescently-labeled antibodies or streptavidin. For protein detection, one may use as capture probes antibodies or derivatives thereof which capture the target proteins and as labeling a second and possibly a third antibody. The streptavidin-biotin pair is also of use for the labeling of the protein arrays.

A second labeling method was recently proposed, based on colorimetry. It makes use of an indirect labeling for protein or nucleic acid molecules and is based on the use of a metallic precipitate which is formed at the location of the target so as to form an easy to detect metallic surface (WO 00/72018). The labeling is indirect making use of a biotin or hapten labeled target and then of gold particles which catalyze the formation of the metallic precipitate.

Other methods have also been proposed, based upon the precipitation of specific products resulting from a colorimetric labeling (US 5,270,167, US 4,731,325, EP-A-0301141) or from an enzymatic activity (EP-A-0393868, WO 86/02733, EP-A-0063810). However, said methods are either characterized by a low sensitivity or are not always adequate for the detection of a target compound upon "hybridization chips".

Other alternative detection methods that present a high sensitivity are described in the documents US-5,821,060 and WO95/04160 and are based upon the detection using expensive devices, such as mass spectrometers.

One problem which arises from such miniaturized and multi-parametric assays is to be able to convert the level of the signals obtained on the different discrete regions of an array into the concentrations of the corresponding targets present in the solution incubated on the array and from there to their concentrations in the biological sample.

The reason for the difficulty is that the signal on each discrete region depends on the concentration of the target in the incubated solution, but also on many other factors such as complexity and efficiency of the labeling, sensitivity of the detection method, settings of the detector and also dynamic ranges of the detection method. Even the best detection method has a limited range of accuracy. Signal measurement which contains usually a linear part, being more or less extended, is followed by a saturation plateau or plateau level.

Different methods of quantification of targets on micro-arrays have been proposed.

Current micro-array analyses rely on normalization and quality control methods that often assume evenly distributed changes, and/or absence of global shifts in gene expression across the array surface. Spotted micro-array features, such as housekeeping genes, sample pools, genomic DNA, or all genes on a micro-array are typically used for normalization. Normalization based on these features is not always appropriate, especially for small focused arrays (versus whole genome micro-arrays), where unbalanced changes are likely to occur, and will have significant effects on the relative hybridization signal intensities between biological samples. As a result, normalization based on such features will give rise to inaccurate interpretations of gene expression data.

According to WO2004/064482, normalization and quality assessment of micro-array data, where unbalanced gene expression is anticipated, can be accomplished by the addition of several different internal controls, non-species nucleic acid targets of different concentrations into the RNA sample of interest prior to labeling and hybridization. Different concentrations of internal control targets are chosen to mimic a broad range of expression profiles. Probes complementary to the internal control targets are printed at equivalent concentrations on the micro-array. Variation between internal control target concentrations in the sample results in different fluorescence intensities detected for each internal control probe. Since detection of the different internal controls will be equivalent between RNA samples, and are not affected by unbalanced or global shifts in gene expression within the RNA sample of interest, they can be used for accurate normalization and interpretation of gene expression data from focused micro-arrays.

De Longueville et al. disclose a similar normalization method based on the use of an internal standard which is spiked at known concentration into the mRNA sample just before the synthesis of labeled cDNA (2002, Biochemical Pharmacology, 64, 137-149). This control is a fragment of HIV-1 sequence which is not related to the target mRNA present in the sample. To maximize the dynamic range of micro-arrays, the same arrays are scanned at different photomultiplier settings of the scanner. Using different gains allows the quantification of both the high and low copy expressed genes. Data mining and determination of significantly expressed genes in the test compared to the reference arrays is performed according to the following method. The spots intensities are first corrected for the local background and than the ratio between a test and a reference arrays are calculated. To take into account variation in the different experimental steps, the data obtained from different hybridizations are normalized in two ways. First the values are corrected using a factor calculated from the intensity ratios of the internal standard in the reference and the test samples. The presence of an internal standard probe at six different locations on the micro-array allows measurement of a local background and evaluation of the micro-array homogeneity, which is going to be considered in the normalization. However, the internal standard control does not account for the quality of the mRNA sample; therefore a second step of normalization is performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained (Chen et al, J. Biomed. Optics 1997, 2: 364-74). Ratios outside the 95% confidence interval are determined to be significantly changed by the treatment.

The drawback to using an internal control, where varying amounts of different internal control are added to the RNA sample of interest, is that it requires accurate measurement of extremely small quantities of those several RNA targets at low concentrations. The technical error associated with measurements at the low range required for micro-array analysis results in unacceptable variation between samples that will have a significant influence on normalization and interpretation of gene expression data. In addition, the optimization and preparation of multiple internal control targets and probes is time-consuming and costly.

US2006/0115840 provides another approach to micro-array normalization and quality control. This document teaches the addition of a constant and accurately measurable quantity of external control target to the biological sample prior to labeling and hybridization combined with the printing on a micro-array of several different concentrations of the external control probe. The external control is non-species nucleic acid or protein target. Variation in the amount of printed probe results in variation of the amount of hybridized external control target resulting in the detection of a broad range of hybridization signal intensities.

WO2006/027088 relates to an alternative method, whereby capture probes being capable of specifically binding to targets (polynucleotides or proteins) contained in sample solution are printed in various concentrations on a support. This concentration curve is used to increase the dynamic recording range for gene detection on micro-array when scanning the arrays at different photomultiplier settings of the scanner.

All the above mentioned normalization methods were applied to micro-array being detected using fluorescent dye and fluorescent scanner.

WO00072018A1 proposes a method for the detection and/or quantification of target compounds on micro-array using a colorimetric method based on the detection of metallic precipitates. As only one picture of the array is taken, there is no possibility to increase the dynamic range.

Accordingly, there is a need for a method and system that allows for more accuracy in the quantification of the data on micro-array.

## Aims of the invention

The present invention aims to provide a new quantification method and system (or means) of a (one or more) target compound present (possibly simultaneously) in a biological sample which does not present the drawbacks of the state of the art.

The present invention aims to provide a quantification method and system (or means) that allows obtaining comparative quantification of different target compounds being at different concentrations within one sample contacted with one array and also from different experiments performed on different arrays.

A last aim of the present invention is to be able to provide a method and system (or means) based upon the use of a target concentration curve from which a target compound within a sample can be quantified on an absolute level.

## Summary of the invention

The present invention is related to a method and system for a quantification of a (one or more) target compound being a polynucleotide (a nucleotide sequence) or a protein (peptide) present in a solution and obtained from a biological sample, by binding to a corresponding capture probe being fixed upon an array, which means that the target amount present in the biological sample is obtained by the method and system of the invention.

Advantageously, said method comprises the steps of:

a) possibly obtaining a dilution (by a known dilution factor) of the target compound(s) initially present in the sample;

b) possibly labeling the target compound(s);

c) putting into contact the target compound(s) with (a) capture probe(s) in order to allow a specific binding between said target compound(s) and said capture probe(s), said capture probe(s) being fixed upon a surface of a solid support according to an array comprising a density of at least 10 discrete regions per $cm^2$, each of said discrete regions being fixed with a species of capture probe (capable of specifically binding a target compound) and wherein at least 4 additional discrete regions are printed with solutions containing known, different and increasing concentrations of a (labeled) detection molecule, said detection molecule being from the same family (either polynucleotide (if the capture probe is a polynucleotide) or protein (if the capture probe is a protein (peptide)) as the capture probes, wherein each of said discrete regions has preferably a diameter comprised between about 50 and about 500 $\mu$m,

d) detecting simultaneously each signals resulting from the binding between the target compound(s) and corresponding capture probe(s) and resulting from the printed detection molecule in the different discrete regions, by the same detection means and in the same conditions of detection (this mean that the nature of the signal is the same, but the value is different),

e) obtaining a detection curve (C) of the detected signals of said detection molecule (4, 4', 4", 4''') in the at least 4 additional discrete regions (B) of the solid support (3) surface as a function of their corresponding printed detection solution concentrations in arbitrary units (AU),

f) converting the signal(s) obtained from the target compound(s) (1) bound to a specific capture probe(s) (2) into a concentration value presented in arbitrary units (AU) by a conversion step upon the detection curve (C) of the detected signal, and

g) quantifying the target compound(s) by converting said concentration value (AU) into a target amount using a target concentration curve.

The present invention is also related to a program executable on a programmable device containing instructions, which when executed performing the steps e) to the step g) or the step d) to the step g) of the method according to the invention above described.

The present invention is also related to a diagnostic and/or quantification system or kit for the quantification of this target compound (being polynucleotide or protein) and being present in a solution and obtained from a biological sample.

This system or kit comprises:

- a solid support with an (one or more) array surface(s) comprising at least 10 discrete regions per $cm^2$, each of said discrete regions being printed with one species of capture probe capable of specifically binding to a target compound, wherein at least 4 additional discrete regions are printed with solutions containing known different and increasing concentrations of a detection molecule, this detection molecule being from the same family (either a polynucleotide or a protein) as the capture probes, wherein each of said discrete regions has a diameter comprised between about 50 and about 500 $\mu$m,

- the program according to the invention which is a software or a support of this software comprising codes means which are programmed to (or a computer or its processor module configured to) :

d) possibly detecting (or collecting) simultaneously each signal resulting from the binding between target compounds and corresponding capture probes and resulting from the printed detection molecule in the different discrete regions by the same detection means and in the same conduction of detection,

e) obtaining (calculating or providing) an equation corresponding to a detection curve (C) of detected and collected signal(s) of said detection molecule (4, 4', 4", 4''')in the at least 4 additional discrete regions (B) of the solid support (3) surface as a function of their corresponding printed detection solution concentrations in arbitrary units (AU),

f) converting the signal(s) obtained from target compound(s)(1) to be quantified (and bound to its specific capture probe) into a concentration value presented in arbitrary units (AU) (on the basis of said provided equation), or by a conversion step upon the detection curve (C) (by reporting this detected signal upon this detection curve) and converting the signal into a concentration value presented in arbitrary units (AU), and
g) quantifying the target compound(s) by converting said concentration value (AU) into a target amount using a target concentration curve.

In the method and system of the invention the conversion of signal(s) could be obtained directly upon the detection curve by the use of a specific software which is able to collect the detection results of the printed detection molecule in the different discrete regions (each concentration of the detection molecule corresponding to a different detection signal).

Furthermore, in the method and system of the invention, the software could be used for calculating the curve of the detected signals according to the concentrations of the detection molecule, the concentration of the detection molecule or provides an equation that may correspond to this curve, said curve being obtained from at least four different concentrations, preferably at least 10 different concentrations or more.

Therefore, in the method and system of the invention, the software could be also used for providing an equation corresponding to said detection curve, which is used for converting easily and immediately any signal obtained from the binding of a target compound (upon its corresponding capture probe) into a specific concentration value by simply reporting this signal value upon the detection curve of the detected signals or incorporate these signals values into the provided equation and selecting the corresponding concentration value. The equation of the curve is preferably a 4-parameter logistic function.

The present invention is also related to a computer program product (software) comprising program code means configured for performing all or part of the steps of the method according to the invention, when said program is run on a computer and interact with the detector and/or reading device.

The present invention is related to a computer program product comprising program code means stored on a computer readable medium and configured for performing the all or parts if the steps of the method according to the invention, when said program product is run on a computer and interact with the detector and/or reading device.

Said means are able to collect the results obtained from said detection and/or quantification device and possibly the information(s) obtained by said reading device, and said means are able to carry out a diagnostic and/or quantification of a specific target compound resulting from the analysis of said results, possibly correlated to the read information(s).

Said means of this computer program product are able to obtain a discrimination between the spots and a possible detected background noise, for instance by the identification of homogeneous parts of an image after having been merged into two classes used as training sets. This discrimination can be enhanced by post-classification contextual filters techniques.

Said means are also able to identify the contour of the spot itself, which will be superposed to the original image and will allow the measure of intensity level of the counted pixels identified in the spot.

The quantification means allow an integration of all pixels intensity present in the spot or a recording the overall level of intensity of the homogeneous parts of the spot.

Furthermore, these means allow a statistical comparative analysis between the spots of each sample and a control or reference standard (standard target compound) or between two or more spots (preferably with a correlation with the recorded information of the solid support). Image correlation could be obtained between the spot image and said standard target compound spot image in order to discriminate spots that are statistically different in one test compared to another. The different targets of a sample which amounts are statistically different from a reference sample represent a pattern of targets typical of the said sample. A pattern of change in gene expression or protein content determined according by the invention is a particular useful embodiment of the invention.

The recorded signal(s) by the detection device and the reading device can be read, processed as electronically computerized data, analyzed by said appropriate computer program product (software) and process to fulfill the present invention.

The present invention will be described in the following detailed description of the invention in reference to the enclosed figures and presented as a non limiting embodiment of the invention.

## Short description of the drawings

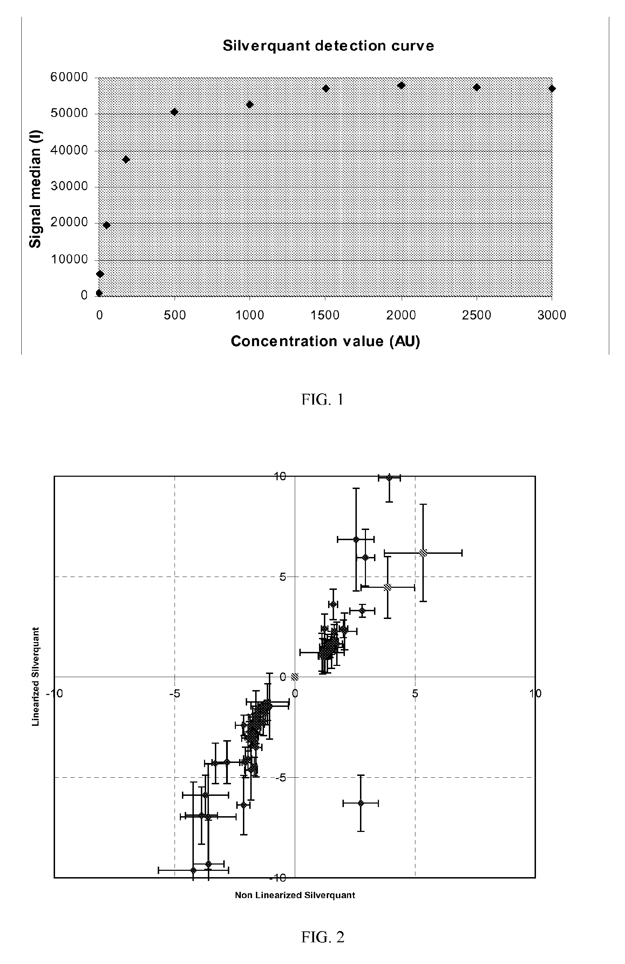

Figure 1 represents a Silverquant Detection curve. Increasing concentrations of a detection molecule (multi-biotinylated polynucleotide) are printed on a glass slide and detected in Silverquant as provided in example 1. The graph is expressed as signal median (I) as a function of the concentration value in arbitrary units (AU) of the printed detection molecule. The biotins of the printed polynucleotide are detected using anti-biotin antibody labeled with gold followed by silver enhancement. The equation of the detection curve is:

$$I = (216.017 - 57136.864) / (1+(C/97.318)^{1.104}) + 57136.864 \text{ with } R^2 = 0.998.$$

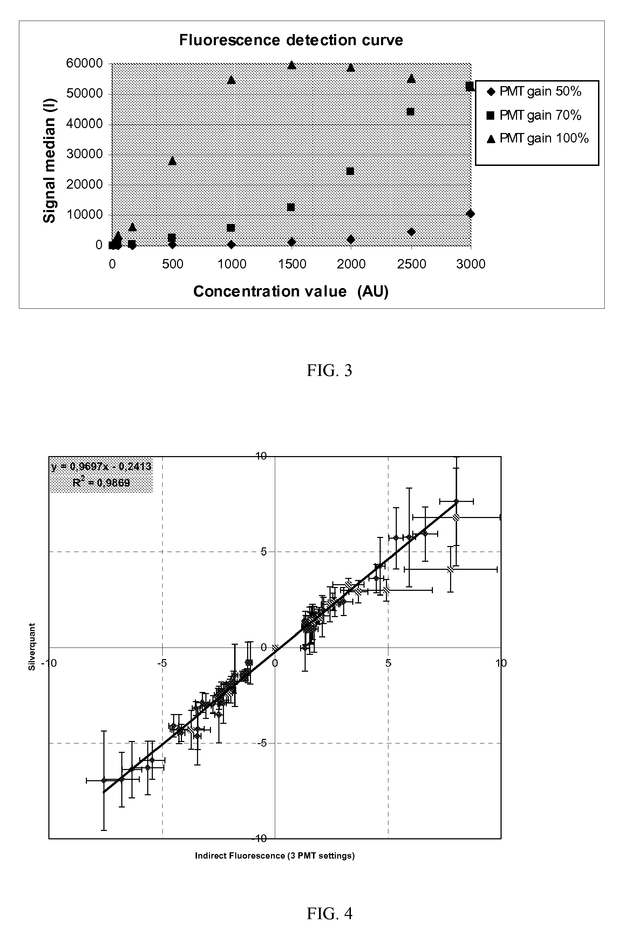

Figure 2 represents the correlation plot between converted Silverquant ratios (y-axis) versus non converted ratios (x-axis) in a gene expression experiment as provided in example 1. The scatter plot represents a zoom on the range [-10; 10].

Figure 3 represents a fluorescence detection curve. Increasing concentrations of a detection molecule (multi-biotinylated polynucleotide) are printed on a glass slide and detected in fluorescence as provided in example 2. The graph is expressed as signal median (I) as a function of the concentration value in arbitrary units (AU) of the printed detection molecule. The biotins of the printed polynucleotide are detected using anti-biotin antibody labeled with Cy3. The detection curve is scanned at three different PMT gains (50, 70 and 100%) to increase the dynamic range for quantification. An equation has been calculated in the linear (non-saturated) part of each of the three curves: PMT 50% (y=8.18X-14831, $R^2$=0.9277), PMT 70% (y=25.313X-22726, $R^2$=0.9746), PMT 100% (y=55.792X-869.161, $R^2$=0.9946).

Figure 4 represents the correlation plot between converted Silverquant® ratios (y-axis) and fluorescence ratios at 3 PMT settings (x-axis) in a gene expression experiment as provided in example 2. The $R^2$ statistic measuring the conformity between ratios for both methods has been computed and corresponds to 0.9869. The scatter plot represents a zoom on the range [-10; 10].

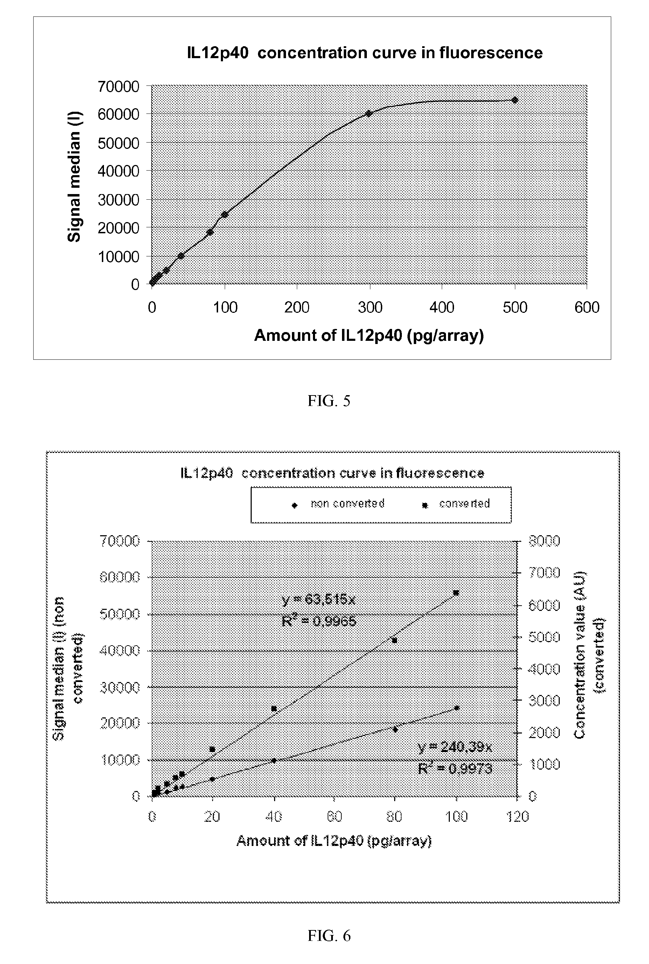

Figure 5 represents a concentration curve of target compound IL-12p40 (protein) in fluorescence as provided in example 3. Different amounts of target compound IL-12p40 ranging from 0 to 500 pg are tested on different arrays (SignalChip Human Cytokine microarray). The curve is expressed as the signal median (I) as a function of the amount of IL-12p40 (pg/array).

Figure 6 represents a concentration curve of target compound IL-12p40 (protein) in fluorescence as provided in example 3. IL-12p40 signals from individual arrays are converted using the detection curve generated from the detected signals of the detection molecule (control antibody) printed on the same array. The target concentration curve is expressed as concentration value (AU) (converted) (right y-axis) as a function of the amount of IL-12p40 (pg/array) (x-axis). The linear regression of the curve is given by the equation: y = 63.515X with $R^2$ = 0.9965. The concentration curve is compared on the same graph with the curve from figure 5 (signal median (I), non converted, left y-axis) . Only the non saturated signals are plotted, i.e between 0 and 100 pg IL12p40/array. The curve of signal median (non-converted) is linear in this concentration range and the linear regression is given by the equation: y = 240.39X with $R^2$ = 0.9973.

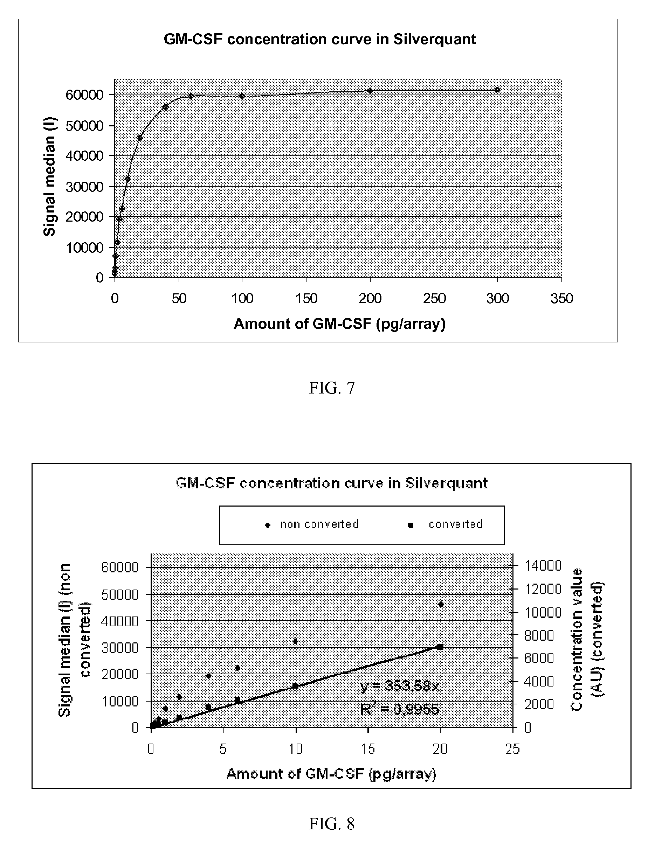

Figure 7 represents a concentration curve of target compound GM-CSF (protein) in Silverquant as provided in example 4. Different amounts of target compound GM-CSF ranging from 0 to 300 pg are tested on different arrays (SignalChip Human Cytokine microarray). The curve is expressed as the signal median (I) as a function of the amount of GM-CSF (pg/array).

Figure 8 represents a concentration curve of target compound GM-CSF (protein) in Silverquant as provided in example 4. GM-CSF signals from individual arrays are converted using the detection curve generated from the detected signals of the detection molecule (control antibody) printed on the same array. The target concentration curve is expressed as concentration value (AU) (converted) (right y-axis) as a function of the amount of GM-CSF (pg/array) (x-axis). The linear regression of the curve is given by the equation: y = 353.58X with $R^2$ = 0.9955. The concentration curve is compared on the same graph with the curve from figure 7 (signal median (I), non converted, left y-axis). Only the non saturated signals are plotted, i.e between 0 and 20 pg IL12p40/array. The curve of non-converted signals is non linear in this concentration range.

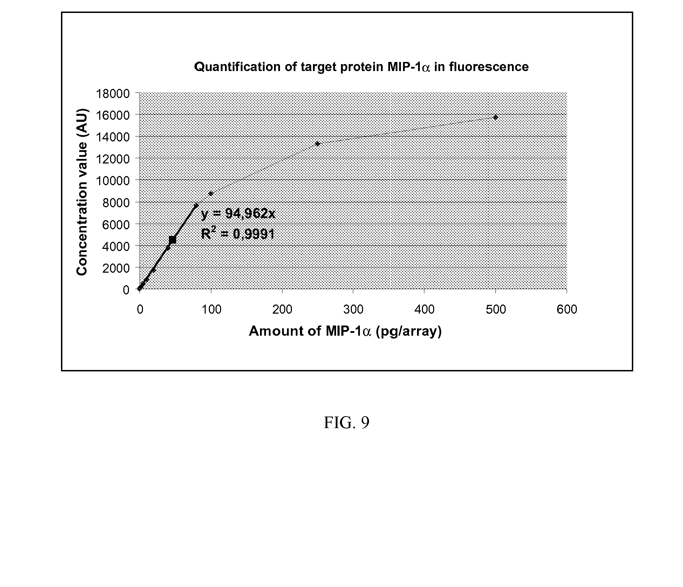

Figure 9 represents a quantification of MIP-1α target protein in fluorescence. A concentration curve of MIP-1α is established as provided in example 5 and in figures 6 and 8. The target concentration curve is expressed as concentration value (AU) (converted) (y-axis) as a function of the amount of MIP-1α, (pg/array) (x-axis). In the linear part of the curve (i.e between 0 and 80 pg of MIP-1α /array) a linear regression of the curve is drawn and the equation is: y = 94.962X with $R^2$ = 0.9991. The concentration value (AU) generated for an unknown MIP-1α amount contained in a test sample is reported onto the target concentration curve (as indicated by a black square) and the amount of MIB-1α is calculated on the basis of the equation and corresponds to 47 pg.

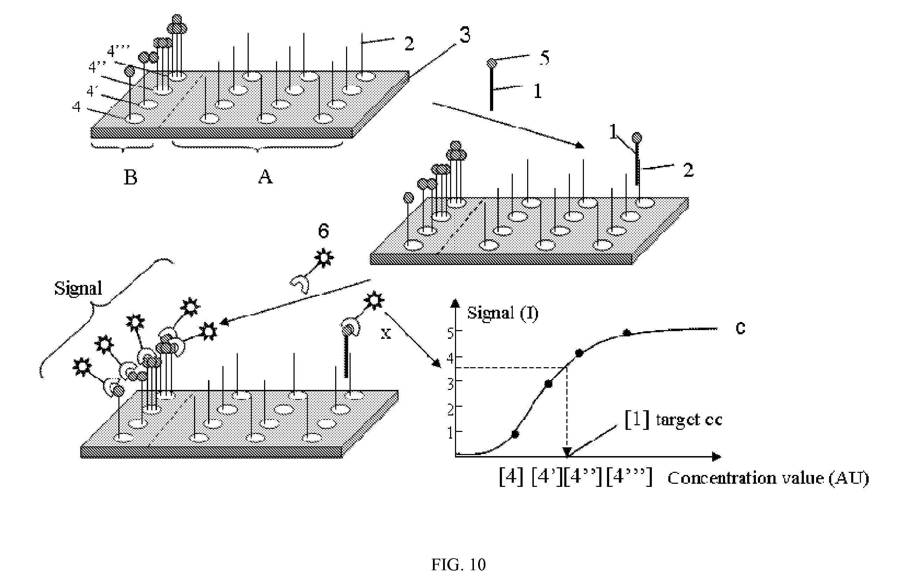

Figure 10 is a schematic representation of the different steps of the method of the invention for polynucleotide quantification. Different capture probes (2) are immobilized in discrete regions (A) of the solid support (3) surface and can bind specifically biotinylated (5) target polynucleotides (1). Increasing concentrations of biotinylated detection molecule are immobilized (4, 4', 4", 4"') in additional discrete regions (B) of the solid support surface. Biotin (5) of both the bound target polynucleotides and the detection molecule is universally detected with an anti-biotin antibody (6) being labeled differently (with another label than biotin). Then a detection curve (c) of the detected signals (I) of the detection molecule versus the printed detection solution concentrations (concentration value in AU) is constructed. Each target polynucleotide (1) signal (x) (obtained from its specific binding to a capture probe) is converted into a concentration value [1] by a conversion step using the detection curve (C). An equation of said detection curve based on 4-parameter logistic function may be used to perform said conversion.

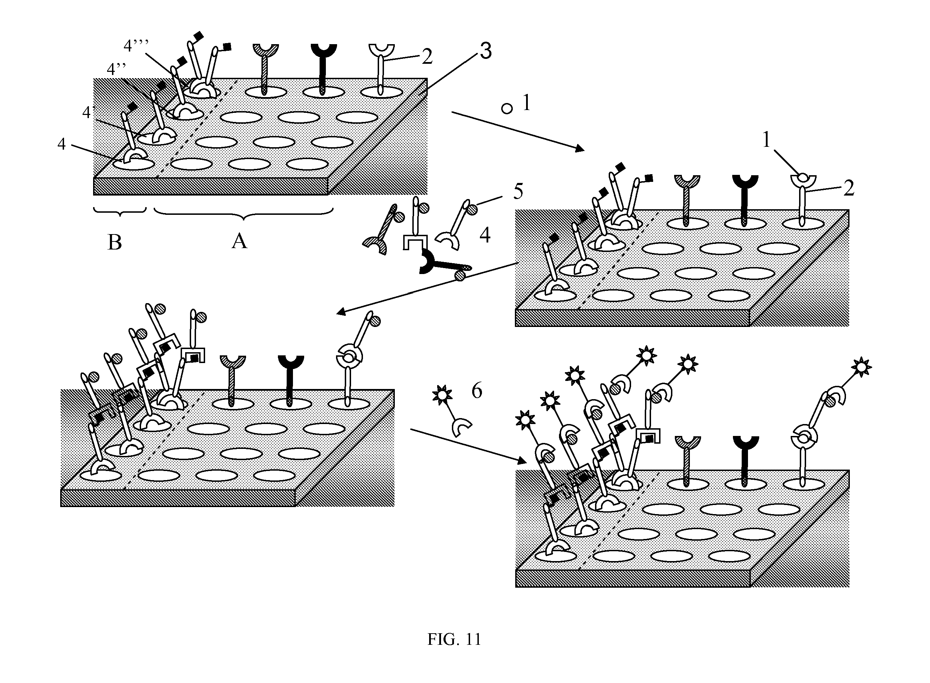

Figure 11 is a schematic representation of the different steps of the invention method for protein quantification. Different capture probes (2) are immobilized in discrete regions (A) of the solid support (3) surface and can bind specifically target proteins (1). Increasing concentrations of detection molecule are immobilized (4, 4', 4'', 4''') in additional discrete regions (B) of the solid support surface. Detection molecule is labeled with an hapten (e.g. HRP). The bound target proteins (1) and the detection molecule are detected using a mix of primary antibodies (4) (labeled with biotin (5)), each one being capable of recognizing a target protein (1) or the hapten of immobilized detection molecule. Then an anti-biotin secondary antibody (6) being labeled is contacted with the array, thus labeling universally discrete regions carrying the biotin (5). The generated signals are treated as provided in figure 10. The ultimate quantification of the amount of target protein requires a conversion of the concentration value (AU) to a protein amount by using a target concentration curve as provided in figure 9.

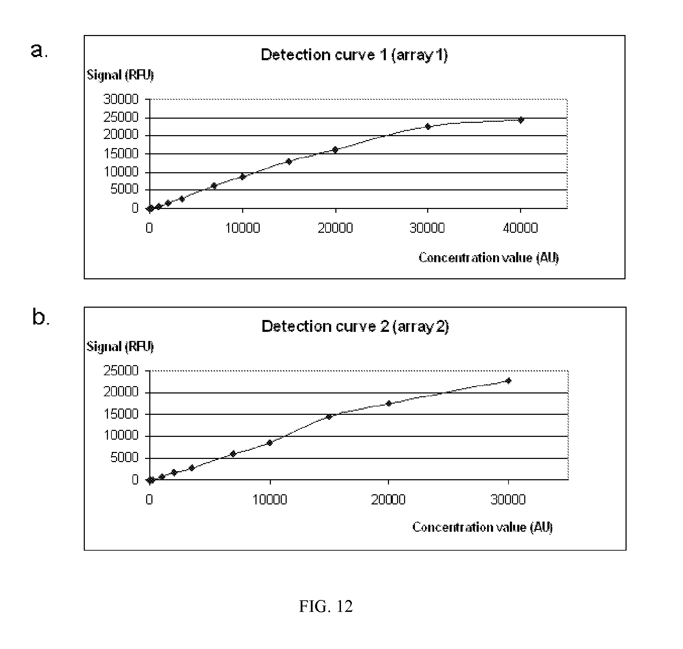

Figure 12 represents detection curves constructed from the fluorescence intensities (signal minus local background, averaged for the triplicate spots) of the positive detection controls in the SignalChip Human Cytokine micro-array where (a) 10 pg of TNF$\alpha$ and IL-1$\beta$ have been incubated (array 1) or (b) 20 pg of TNF$\alpha$ and IL-4 (array 2) .

> RFU: relative fluorescence units
> AU: arbitrary units corresponding to the concentration values of the positive detection controls.

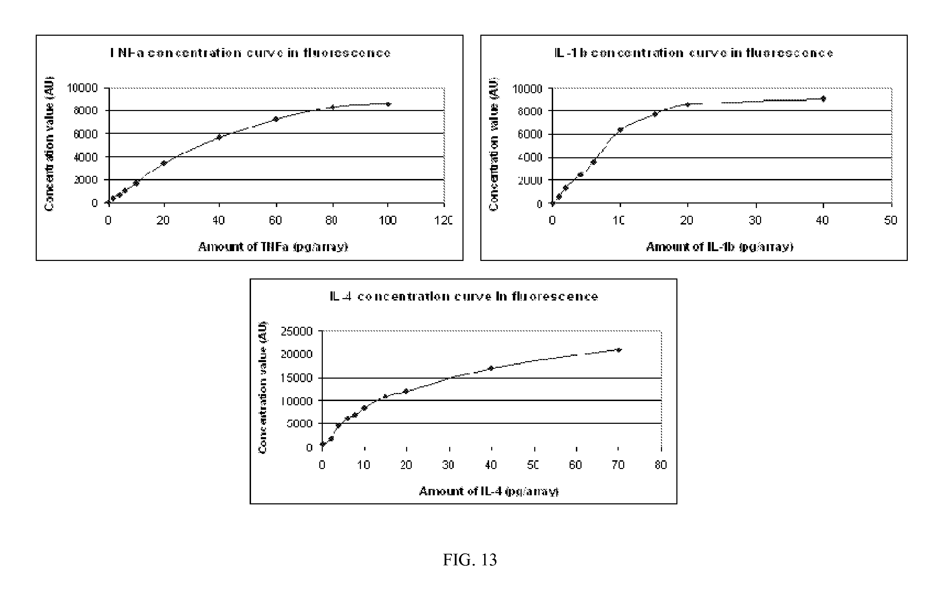

Figure 13 represents target concentration curves in fluorescence for TNF$\alpha$, IL-1$\beta$ and IL-4 polypeptides.

Table 1 represents converted and non converted silverquant ratios of 7 genes (FN, ON, APOJ, FMO5, CAS7, PEPT1, PXR) generated by a gene expression experiment on micro-array as provided in example 1. The table includes a comparison of the ratios with those obtained by Real-time PCR.

Table 2 provides a comparison of the simultaneous quantification of three cytokines (TNF$\alpha$, IL-1$\beta$, IL-4) (example 6). These cytokines are present at a similar concentration (10 pg or 20 pg) in the sample but they show very different signal intensity (RFU) on the array, the difference in signal intensity being a factor of 5 between TNF$\alpha$ (10 pg) and IL-1$\beta$ (10 pg) and a factor of about 3.5 between TNF$\alpha$, (20 pg) and IL-4 (20 pg) . By converting the signal intensity obtained for each cytokine into concentration value (AU) followed by the conversion of the AU (arbitrary units) into cytokine amount (pg) based on the concentration curve of the individual cytokines, the initial concentration of the two cytokines was restored.

## Detailed description of the invention

"Detection molecule" refers to a molecule that does not hybridize with or bind specifically to the target compound (s) of the sample under study. In a preferred embodiment, a polynucleotide is a detection molecule if its complement is not present in the target compound(s) present in the sample. The target compound(s) are preferably a plurality of polynucleotides or proteins to be hybridized or bound specifically to an array.

The "chips or array" according to the invention are any kind of solid support that allow the formation of (micro-) arrays of capture probes (specific pattern) upon one or more of its surfaces. Said solid support can be made of glass, filter, electronic device, polymeric or metallic material, etc., including material such as plastic support. Preferably, said arrays contain discrete regions or specific locations (advantageously presented according to a specific pattern), each of them containing only one species of capture probe.

The term "only one species" of capture probe means that the capture probe sequence (in nucleotide bases for a polynucleotide or in amino acids for a protein) is substantially the same in a given discrete region. Substantially means that more than 90% of the capture probe sequence is the same (length and composition).

The term "family" of capture probe means that the capture probe is either a polynucleotide or a protein. The detection molecule is from the same family as the capture probe means that if the capture probe is a protein (or peptide), the detection molecule is also a protein (or peptide) and if the capture probe is a polynucleotide (or a nucleotide sequence) the detection molecule is also a polynucleotide (or a nucleotide sequence).

The inventors have unexpectedly found that it was possible to obtain by a very simple method, a quantification of a (one or more) target compound 1 (being a polynucleotide or a protein) present in a sample solution and incubated on an array made of capture probes 2 upon a sold support surface 3 even when the detection method did not show a linear relationship between the target compound 1 concentration and the detected signal. In a preferred embodiment, the quantification of the target compound 1 is performed from signals which are not in the linear part of the detection curve. This is particularly useful when using a colorimetric detection method for which the detection curve is mostly non linear. However, signals at saturation are never usable for quantification whatever the detection method used.

As shown on figure 10 and 11 the solution is obtained by providing a detection curve of detected signals obtained from a detection molecule (4,4',4'',4''') fixed at different concentrations upon the solid support surface 3.

Advantageously, the signal of detection of the different fixed molecules (detection molecules and target compound) is performed simultaneously upon the different location of (A, B) of the solid support surface 3.

Advantageously, this signal is obtained with the same detection means with the same condition, which means that the characteristic of signal is the same, but is value is different.

The volume of printed solutions of at least four fixed detection molecule (4, 4', 4", 4"') concentrations can be comprised between about 0.01 and about 5 nl. These values correspond to volume ranges used for micro-array fabrication using an arrayer.

The detection molecule is printed in discrete regions with a solid pin or alternatively can be deposited on the surface of a solid support in the form of a droplet.

The detection curve of detection molecule of the array can be obtained by printing at least 10 different concentrations of detection molecule solution in different discrete regions.

The concentration of the printed detection molecule solution can be comprised between about 0.3 and about 300 nM for a detection molecule being a polynucleotide. Preferably the concentrations are about: 0.3, 1, 5, 17.5, 50, 100, 150, 200, 250, and 300 nM.

The concentration of the printed detection molecule solution can be comprised between 0.025 and 50 $\mu$g/ml for a detection molecule being a protein. Preferably the concentrations are about: 0.025, 0.050, 0.075, 0.10, 0.35, 0.70, 1, 3.5, 7, 10, 15, 20 and 50 $\mu$g/ml.

The different concentrations of the printed detection molecule solution can spread over at least 2 log concentration, preferably over at least 3 log concentration.

The factor separating the different concentrations of the printed detection molecule solution can be comprised between about 1.2 and about 5.

Advantageously, the quantification of the target compound is increased by at least 1 log compared to the quantification in linear part of the detection curve.

The fixation of capture probes can be obtained by linkage of amino groups on activated glass of the solid support bearing aldehyde moiety.

Such method avoids the burden and limitations due to the use of internal standards or complex mixture to be added to the target sample. In this method the target solution does not require any additive or special treatment. The unexpected finding is that the signal of the target compound which goes to the process of hybridization and labeling on the array can be converted for quantification according to signals obtained from different concentrations of printed detection molecule solutions and such conversions are valid in both the linear and non linear part of the signal detection curve.

The same method is used for the construction of a target concentration curve (standard target curve) where different amounts of a target compound, being preferably a purified polynucleotide (nucleotide sequence) or protein (peptide) are incubated and quantified on different arrays.

The target concentration curve can be generated by contacting different amounts of target compound upon different arrays and converting the signal obtained into concentration value. The number of different concentrations contacted upon different arrays is at least 3, preferably at least 6 and more preferably at least 8.

Moreover the present invention allows the conversion of the detection signal of a target compound on different arrays as long as the targets are treated and detected in the same manner. Such conversion allows combining the results of the different arrays and obtaining a concentration curve of the targets in a linear form. The slope of this curve is mostly dependant on the incubation and reaction conditions of the target with its capture probes. If the conditions are kept constant a coefficient is calculated from such target concentration curve in order to convert the target concentration value (AU, converted) into a target amount. If the condition of incubation changed from one assay to the other, the slope of the curve changes but given the fact that the present invention allows obtaining a linear concentration curve, a minimum of one assay with one concentration of target is enough to determine the slope coefficient and calculate the amount of the unknown target in the different samples to be analyzed.

It is thus not necessary to perform a full concentration curve of target compounds in order to perform the quantification.

A coefficient can be obtained (calculated) for the slope of the target concentration curve. In another embodiment, a coefficient of the slope of the target concentration curve is obtained by the assay of only one given target amount performed on one array.

The quantification of the target compound in the sample solution can be obtained (calculated) from the target concentration curve by converting the target amount into target concentration in the sample solution. The conversion may be obtained using an equation of the target concentration curve being non linear. This method is particularly useful when the linear part of the curve (after conversion) is very short and limited to low amounts of target (which are far from the saturation). The use of an equation being non-linear allows taking into account data which are in the non-linear part of the curve for higher amounts of target as long as the saturation is not reached. As a consequence the dynamic range for quantification is increased as compared to an equation being a linear regression.

The quantification of the target compound in the sample solution can be obtained (calculated) from the coefficient of the slope of the target concentration curve.

The method of the invention is preferably performed on two different samples, and the ratio between the concentrations of a target compound in the two sample solutions is obtained by calculating the ratio of the concentration value (converted from the signal intensity) obtained on the two arrays.

The amount of the target compound in a sample solution is the amount of the target compound in the array incubated solution corrected by a dilution factor of the said solution compared to the original sample solution.

The detection molecule and the capture probes can be (possibly labeled with biotin) double stranded DNA molecules able to bind to protein being transcriptional factors.

The signal is obtained by a binding of their transcriptional factors and labeled double stranded DNA with specific primary antibodies.

Secondary antibodies are used for a (preferably simultaneous) detection of these fixed primary antibodies.

In a preferred embodiment, the target compound is a cytokine protein selected from the group consisting of: IL-1α, IL-1β, IL-1ra, IL-2, IL-4, IL-6, IL-8, IL-10, IL-12 p40, IL-12 p70, IL-17, TNFα, TNF-RI, TNF-RII, IFNγ, GM-CSF, Eotaxin, MIP-1α, MIP-1β, Rantes.

Advantageously, the method of the invention allows the simultaneous quantification of at least two cytokines having different detection sensitivity. Different detection sensitivity means that a similar amount of two cytokines in a sample solution gives after binding to the capture probes different signals, one high and one low. The difference in detection signals may be a factor of at least 2, or at least 5. By converting the signal intensity obtained for each cytokine into concentration value (AU) followed by the conversion of the AU into cytokine amount (pg) based on the concentration curve of the individual cytokines, the initial concentration of the two cytokines is restored. In a preferred embodiment, the method of the invention allows simultaneous quantification of at least two proteins (peptides) differing in detected (measured) signal by a factor of at least 2 when present at the same amount.

The fixation (binding) of DNA strands on proteins thereafter specifically attached to sites specific locations on a substrate, is described in the document US-5,561,071. It is also known that capture chemicals can be linked to microtubes that are then spatially arranged in order to produce an array, as described in the document GB-3 319 838, or to obtain the direct synthesis of oligonucleotides on specific surfaces by using photolithographic techniques as described in the documents WO97/29212 and US-5,632,957.

All these methods for the fixation (binding) of capture probes on the surface of a solid support in order to obtain the above-described arrays are compatible with the present invention.

The biological target compounds according to the invention may be present in a biological (or possibly a non-biological) sample such as possibly purified clinical samples extracted from blood, urine, feces, saliva, pus, serum, tissues, fermentation solutions or culture media. Said target compounds are preferably isolated, purified, cleaved, copied and/or genetically amplified, if necessary, by known methods by the person skilled in the art, before their detection and/or quantification upon the "hybridization chips".

Detectable labels suitable for use in the present invention include any composition detectable by electromagnetic light emission. In an embodiment, the signal obtained on the different discrete regions of the array is fluorescent. The fluorescent label can be incorporated into the target by enzymatic or chemical reaction. Typical enzyme reaction includes the incorporation of nucleotide analogues into the target. Alternatively, primers labeled at their 5' end with a fluorescent dye can be incorporated into the target.

Fluorochromes can also be incorporated into the targets by chemical reaction such as the reaction of fluorescent dye bearing a N-hydroxysuccinimide (NHS) group with amines groups of the targets. Useful fluorescent dyes in the present invention include cyanine dyes (Cy3, Cy5, Cy7), fluorescein, texas red, rhodamine, green fluorescent protein. Preferably, the excitation wavelength for cyanin 3 is comprised between 540 and 558 nm with a peak at 550 nm and the emission wavelength is comprised between 562 and 580 nm with a peak at 570 nm.

Preferably, the excitation wavelength for cyanin 5 is comprised between 639 and 659 nm with a peak at 649 nm and the emission wavelength is comprised between 665 and 685 nm with a peak at 670 nm. Preferably, the excitation wavelength for cyanin 7 is comprised between 733 and 753 nm with a peak at 743 nm and the emission wavelength is comprised between 757 and 777 nm with a peak at 767 nm.

Patents teaching the use of such labels include U.S. Patent Nos. 3,817,837; 3,850,752; 3,939,350; 3,996,345; 4,277,437; 4,275,149; and 4,366,241.

Preferably, the signal obtained on the different discrete regions is a precipitate, preferably a metallic precipitate. In a preferred embodiment, the metallic precipitate is obtained by chemical reduction of silver in the presence of colloidal gold particles coupled to the bounded target compound. In an alternative embodiment, the metallic precipitate is obtained in the presence of an enzyme. Advantageously, a reduction of silver in the presence of colloidal gold allows the formation of a precipitate (metallic deposit) at a distance not exceeding few micrometers from the bounded target compound to its corresponding capture probe.

According to the invention, the specific locations on the array are smaller than 1000 μm in length.

These locations or spots have preferably a diameter comprised between about 10 and about 500 $\mu$m and are separated by a distance of similar order of magnitude, so that the array of the solid support comprises between about 100 and about 250,000 spots upon the surface of 1 cm$^2$. However, it is also possible to prepare spots smaller as 1 $\mu$m or less upon which the capture probes are fixed. The formation of said spots or locations would be obtained by known microelectronic or photolithographic processes and devices that allow the fixation (binding) of said capture probes on the surface of the solid support either by a covalent linkage or a non-covalent adsorption. The covalent linkage technique is preferred in order to control specifically the sites of capture probes fixation and avoid possible drawbacks that may result with several capture probes (like nucleic acids or antibodies) that can be desorbed during incubation or washing step.

Preferably, the fixation (binding) of biological molecules like proteins, peptides or nucleic acid sequences or even sugar by linkage of amino groups on activated glass (solid support) bearing aldehyde moiety. The incorporation of an amine group in the nucleic acid chain is easily obtained using aminated nucleotide during their synthesis. Aminated amino acids can be fixed upon the surface of a solid support like glass bearing aldehyde groups as described by Schena et al. (Proc. Natl. Acad. Sci. USA, 93, pp. 10614-10619 (1996)) or as described in the document US-5,605,662 and the publication of Krensky et al. (Nucleic Acids Research, 15, pp. 2891-2909 (1987)). The linkage between an amino and a carboxyl group is obtained by the presence of a coupling agent like carbodiimide compounds as described by Joos et al. (Anal. Biochem., 247, pp. 96-101 (1997)). Aminogroups also form covalent links with other chemical reactive groups such as epoxide, acrylate, alkyl halide,acylhalide, isocyanate or thiocyanate. Thiol modified oligonucleotides can be used also to obtain a reaction with amino groups upon the surface of a solid support in the presence of crosslinking molecules (Chrisey et al., Nucleic Acids Research, 24, pp. 3031-3039 (1996)). Similarly, oligonucleotides can be fixed to a gel like polyacrylamide bearing hydroxyl and aldehyde groups as described in the document US-5,552,270 and WO98/28444. Sugars such as polysaccharides or sugar bearing proteins are best fixed after periodate oxidation into dialdehyde and then fixation on aminated surface.

Polyvinyl or polyacrylic polymers bearing or containing in the resin chemical reactive goups such as aldehyde, epoxide, acrylate, hydrazine, thiocyanate are also an embodiment of this invention. One particular useful method is the grafting or coating of a polyacrylate polymer containing aldehyde groups by incorporation of glycidyl methacrylate such as described by Eckert et al (Biomaterials, 2000, 21, 441). Polymers bearing reactive groups are possibly coated on any surfaces such as glass, metal or plastic making then available as microarray supports.

Polymers such as polyolefine, polyvinyl, polyacrylique, polymethylmethacrylate bearing or containing in the resin chemical reactive goups such as aldehyde, epoxide, acrylate, hydrazine, thiocyanate are also an embodiment of this invention. Polymers bearing reactive groups are possibly coated on any surfaces such as glass, metal or plastic making then available for micro-array supports. Of particular interest is the use of spin coating and radcure radiation for the formation of a polymer onto the surface of the support while incorporating chemicals with reactive groups for capture probe fixation. One of such chemicals is epoximethacrylate which incorporates into the polymer chain through is vinyl group but keep its epoxide group reactive for the further fixation of capture probes.

According to a preferred embodiment of the present invention, the binding of the capture probes upon the surface of the solid support is obtained according to the method described in the document WO02/18288 incorporated herein by reference.

The binding (or recognition) of the target compounds upon corresponding specific capture probes may be a spontaneous non-covalent reaction when performed in optimal conditions. It involves non-covalent chemical bindings. The medium composition and other physical and chemical factors affect the rate and the strength of the binding. For example for nucleotide strands recognition, low stringency and high temperature lower the rate and the strength of the binding between the two complementary strands. However, they also very much lower the non specific binding between two strands (which are not perfectly complementary). When several sequences are similar, the specificity of the binding can be enhanced by addition of a small amount of non-labeled molecules, which will compete with their complementary sequence, but much more with the other ones, thus lowering the level of cross-reactions.

The optimization of the binding conditions is also necessary for antigen/antibody or ligand/receptors, chemical-enzymes recognition, but they are usually rather specific.

In a particular embodiment, the target compound is identified and/or quantified according to a signal characteristic of cell activation. Cell activation include a large range of processes among which phophorylation, acetlylation or methylation leading to the presence of phophate, acetyl or methyl groups on proteins, DNA or sugars. The presence of these groups is best obtained by the use of antibodies specific of the presence of such groups in particular locations of the proteins, DNA or sugars.

In another embodiment, the detected target protein is detected after interaction with another molecules bound to the support either directly or through another molecule. Of particular interest is the use of antibodies to immobilize one particular protein and to screen for the presence in a sample for other proteins which interact with the immobilized first protein.

A preferred embodiment of this invention is to take advantage of the amplification given by the catalytic reduction of Ag$^+$ in the contact of other metals like gold. Gold nanoparticules are currently available and they can be easily fixed (bounded) to molecules like protein. For example, streptavidin and antibodies coated gold particles are available on the market (BBI International, Cardif, England).

According to a preferred embodiment of this invention, the method and system of the invention are based upon the use (uses) of a labeled (5) target compound (1), which is then recognized by a conjugate (6) bound to a reporter molecule resulting in a detectable signal. This label (biotin, hapten, ...) can be considered as a first member (5) of a binding pair. For DNA, the labeling is easily done by incorporation of biotinylated nucleotides during their amplification. For the RNA, biotinylated nucleotides are used for their copy in cDNA or thereafter in the amplification step. Amplification of the nucleotide sequences is a common practice since the target molecules are often present in very low concentrations. Proteins are easily labeled using NHS-biotin or other reactions. Once the labeled (biotinylated) molecules are captured, a streptavidin-gold complex,or an antibody detected against biotin which is the second member (6) of the binding pair, is added and the streptavidin specifically recognizes biotin (5), so that the complex is fixed at the location where the target compound (1) is fixed (bound to its capture probe (2)). If haptens are used as label, an antibody-gold complex will be used. (see figure 10 and 11)

In a specific embodiment, the method and system of the invention is based upon the use (uses) of biotinylated target molecules or reagents recognized thereafter by specific antibodies-gold complex. Then a reactive mixture containing Ag+ and a reducing agent is added on the surface and Ag layers will precipitate on the gold particles leading to the formation of crystal particles. Hydroquinone is the preferred reducing agent for metal precipitation but other reducing agents used in the photographic process are other choices to form silver crystals.

Direct labeling of the target molecules with gold is possible by using gold-labeled antigens, antibodies or nucleotides.

An alternative is to avoid any labeling of the target molecule, and then a second nucleotide sequence is used which is labeled. They then form a sandwich hybridization or a sandwich reaction with the capture probe fixing the target molecule and the labeled nucleotide sequence, which allows the detection to go on. Like above, the labeled nucleotide sequence is able to catalyze itself the precipitation of the metal or it does it through a second complex.

The Ag precipitation corresponds to the location of the binding of biotinylated nucleotide sequence. As said location is well defined, it is possible to identify the presence of said precipitate (specific spot of the array).

The precipitate has the form of small crystals that reach with time a diameter of about 1 $\mu$m. The formation of these small crystals represents a real amplification of the signal since they originated from the presence of gold particles a few nm in diameter.

Because of its granular form, the precipitate advantageously modifies the reflection, transmission, (diffusion) diffraction (scattering), or absorption of the light which is recordable by known detection means. Such transmission (diffusion) assays are typically detected and recorded from the reflection of a light beam with photodiodes. One unexpected observation is that the assay for the presence of silver crystals was found unexpectedly very sensitive.

As a metal, silver is able to reflect light by itself. Because of its metal nature, other methods like variations of an electromagnetic field electric conductance or heat detection (WOO1/85978) are also possible.

In a preferred embodiment, the present invention is related to the use of detector for imaging the sample comprising metallic precipitate by measurement of the absorption of the transmitted light through the surface of the solid support bearing the said metallic precipitate and correlating the said absorbed light with the presence of target molecules fixed on the capture probe present on the surface. The detector preferentially detects in a statistically significant way concentrations of 3 logs or more.

The present invention is also related to a device (integrated in the system of the invention)for imaging a sample preferably integrated in the apparatus according to the invention as a detection and quantification device of precipitate above-mentioned.

The absorbed light in the locations of the capture probes (spots) is preferentially corrected for the background by subtracting the absorbed light in the surface locations not having capture probes preferentially the quantification of each spot is corrected by absorbance of the surface surrounding each spot.

The person skilled in the art is also able to provide means for performing the various steps of the present invention, especially the transformation and the conversion of the measure into a digital form or a set of digital forms by using known means or methods such as the ones existing in software and computer technologies.

**Description of a specific mode of software for obtaining an equation of the printed detection curve.**

After image quantification, and raw data processing (background subtraction, non specific hybridization/binding removal, outliers removal), an equation of the curve expressing the relation between the signal intensities (16-bits greyscale) and the concentrations of the detection controls (concentration scale) is computed.

Computing is performed preferably in the following way:

Nomenclature: a detection control I is noted Pi and has the following coordinates Pi (Ci; Ii), where Ci is the concentration of the solution used to produce this spot and Ii is the measured intensity on the 16 bits greyscale.

1- The first step is the determination of the origin of the curve. The most simple and preferred method is to set the origin point at (0;0). The second method is to set the 0 at a concentration of 0 and a greyscale being the mean of all the negative detection controls of the array. For both methods this 0 is added to the couples of experimental points used to build the curve.

2- The second step is to perform the computing of the detection curve.

In the first preferred embodiment, a logistic regression is performed. The 4-parameter logistic function has been demonstrated to be a very good fitting function of the relation intensity/concentration response.

A typical equation of the curve is:

$$I= - (A1+A2) / (1+(C/X0)^p) + A2$$

Where

- A1 is the value determined at step 1
- A2 is the asymptotic value of the curve
- X0 is the center
- P is the power (growth speed) determining the general shape of the curve.

A1, A2, X0 and p are the 4 parameters defining the logistic curve.

The 4 parameters are computed using the Levenberg-Marquardt's iterations. The iterations are performed until the stability of the parameters reached the defined level.

A regression coefficient $R^2$ is computed for the fitted curve.

In a second embodiment, the computing of the curve is performed using a piecewise linear method on the detection signals. The experimental points are sorted by increasing concentrations. Starting with the zero computed above, the points are taken 2 by 2. These 2 points Pi and Pi+i for a straight line whose equation is given by:

$$I = a C + b$$

With

$$a= (Ii+1 - Ii)/(Ci+1 - Ci)$$

$$b = (Ii.Ci+1-Ci.Ii+1)/(Ci+1 - Ci)$$

A quick improvement of this algorithm is to disregard a certain point Pi when a < 0 in order to obtain a bijective function. (ie for one intensity I corresponds only ONE concentration C).

3- Each signal intensity I of the target compounds of the array is converted to a concentration on the basis of the detection curve or the equation: for each signal I, a concentration C is calculated.

In a first preferred embodiment using the 4-parameters logistic curve, the measured signal intensity of a target compound may exceed the asymptotic value. In this case, the concentration cannot be computed and the infinity value is assigned as concentration for said target compound.

In the second specific embodiment using the piecewise linear curve, the signal intensity of a target compound might be higher than the highest detection point of the curve. An extrapolation of the last segment of the curve is used to compute the concentration of said target compound.

Preferred embodiments of the present invention will be described in the following non-limiting examples in reference to the enclosed figures.

## Examples

Example 1: quantification of gene expression on arrays with Silverquant® labeling and conversion using the detection curve

### *Total RNA samples*

Total RNA of two human tissues (liver and small intestine) were purchased from Ambion®. In order to assess the integrity, analysis of these samples was carried out by capillarity electrophoresis using on Agilent® 2100 BioAnalyser® (Agilent) .

### *Gene expression analysis on DualChip*

For this study, the Eppendorf DualChip® human hepato version 1.0 was chosen. Each DualChip® human hepato consists of two microarrays on one slide that has already been covered with a hybridization frame.
The DualChip® human hepato contained 151 capture probes specific for 151 human genes.

### *Synthesis of labeled target cDNA*

RNA from human small intestine was used as the test sample and RNA from human liver was used as the reference sample. 10 $\mu$g of total RNA from human small intestine or human liver (1 $\mu$g/$\mu$l, Ambion) were mixed with 1 $\mu$l oligo dT Primer (1.5 $\mu$g/$\mu$l, Eppendorf, Germany), 2 $\mu$l of a 10X dNTP mix, made of dATP, dTTP, dGTP (5 mM each, Roche), dCTP, dATP, (800 $\mu$M, Roche), and Biotin-11-dCTP, Biotin-11-dATP (800 $\mu$M, NEN) and 2 $\mu$l of a mix solution of 6 different internal standard poly(A+) RNAs. They served as internal standards to assist in quantification and estimation of experimental variation introduced during the subsequent steps of analysis. After an incubation of 5 min at 65°C and 5 minutes on ice, 9 $\mu$l of reaction mix were added. Reaction mix consisted in 2 $\mu$l RT plus Buffer 10X (Eppendorf, Germany), 1 $\mu$l Prime Rnase Inhibitor Solution (Eppendorf, Germany), 1 $\mu$l cMaster RT enzyme (15 U/ml, Eppendorf, Germany). Reaction mix was added on ice and then incubated at 37°C for 90 minutes. Addition of cMaster RT enzyme and incubation were repeated once. The mixture was then placed at 85°C for 5 minutes. The biotinylated cDNA, was kept at -20°C. This reverse transcription reaction was repeated twice to have enough cDNA.

### *Hybridization of biotinylated-labeled cDNA*

The hybridization and detection were performed using two arrays on the same slide. The left array was hybridized with the test cDNA and the right array with the reference cDNA. Two replicates of hybridization were obtained on two separate slides. The micro-array used in this study was the DualChip human hepato (Eppendorf, Germany). The array comprised 151 selected markers involved human toxicity and also several controls dispensed at different discrete regions on the micro-array. Each capture probe of the array was present in triplicate.
Controls included internal standards, negative detection control, positive and negative hybridization controls. The array also contained 10 different discrete regions which were printed with multi-biotinylated DNA from solutions having the following concentrations: 0.3, 1, 5, 17.5, 50, 100, 150, 200, 250, 300 nM. These concentrations were equivalent to the following concentrations in arbitrary units (AU): 3, 10, 50, 175, 500, 1000, 1500, 2000, 2500, and 3000.
Hybridization mixture consisted of 20 $\mu$l biotinylated cDNA (the total amount of labeled cDNA), 10 $\mu$l HybriBuffer A (Eppendorf, Germany), 40 $\mu$l HybriBuffer B (Eppendorf, Germany), 10 $\mu$l positive hybridization control, 5 $\mu$l Silverquant hybridization additive (Eppendorf, Germnay) and 15 $\mu$l H$_2$O. Hybridization was carried out overnight at 60°C.
The slide was then washed 4 times for 2 min with washing buffer (Eppendorf, Germany).

### *Colorimetric detection*

The slide was incubated for 45 min at room temperature with anti-biotin labeled with colloidal gold diluted in preblocking reagent (Eppendorf, Germany). The slides were then washed 5 times for 2 min with washing buffer and then rinsed for 1 min in Rinsing buffer (Eppendorf, Germany). Slides were then incubated for 5 min with silver enhancement reagent Silverquant® (Eppendorf, Germany), rinsed twice with distilled water and dried.

### *Scanning and quantification*

The array was scanned by transmission measurement using the Silverquant® Scanner (Eppendorf, Germany) and analysed with the Silverquant® analysis software (Eppendorf, Germany). Using the Silverquant® analysis software, the silver intensities of each DNA spot (median intensity of each pixel present within the spot) was calculated using local mean background subtraction. A signal was considered as detected if the median intensity after local background subtraction was at least 2.5 times higher than the local background standard deviation and higher than the mean of the negative hybridization controls plus 2 times their standard deviation. Very bright element intensities (saturated signals, highly expressed genes) were deemed unsuitable for accurate quantification because they underestimated the intensity ratios. They were excluded from quantitative analysis. The intensity value of this saturation level was based on the spotted positive detection control curve on each array.

As each gene was spotted in triplicate, first selection of the data allows averaging the 3 single spots value for each gene. In case of outliers due to local microarray problems, they were removed from the analysis.

*Silverquant® Conversion*

Due to the non linear Silverquant® detection, the first step required was a conversion of the data. An algorithm of curve fitting was applied to the positive detection curve spotted on each array. Then each gene signal was converted into 'concentration units' using the fitted curve.

These steps are detailed hereafter.

Figure 1 is a representation of the detection curve obtained in this experiment. The graph is expressed as signal median (I) as a function of the concentration value in arbitrary units (AU) of the printed detection molecule. The detection curve comprises a non linear first part of the curve ranging from 3 to 1000 AU and a saturated (non linear) second part ranging from 1000 to 3000 AU. The 10 concentrations of the detection curve have been chosen such as to cover equally the concentration range of 3 to 3000 AU.

Then, the software computed an equation to fit a four-parameter logistic curve to these experimental points. In figure 1, the equation is $I = (216.017 - 57136.864) / (1+(C/97.318)^{1.104}) + 57136.864$ with $R^2 = 0.998$.

Once the equation was computed, the signal intensity of each gene was converted into 'concentration' value on the basis of this equation. These values were in AU (arbitrary units). Once every gene has been converted, all values used in the software are in this 'concentration' scale. High signal values were extrapolated by the curve.

The conversion into concentration value aimed to linearize the first part of the curve which was non linear before conversion and thus to extend the dynamic range for quantification. The second aim of the conversion was to be able to compare detection signal of a target compound on different arrays as long as the targets treated and detected in the same manner. Such correction allowed combining the results of the different arrays and obtaining a concentration curve of the targets in a linear form.

*Normalization and data analysis*

To account variation in the different experimental steps, the data obtained from different hybridizations were normalized in two ways. The first step was a local normalization by the internal standard (IS). The array was divided into six zones, each containing two different IS capture probes. These IS capture probes corresponded to different RNA spiking standard concentrations (low and high) of the internal standard mix to ensure the acceptable expression of at least one IS per zone. This special design allowed the computation of a local normalization factor for each zone using the acceptable IS of the corresponding zone in the reference and experiment samples. After calculating the local normalization factor, the ratios for each gene between the test and reference samples in that local zone were corrected by the local normalization factor.

After IS normalization, the next step was housekeeping gene normalization. IS did not actually correct differences in the quality and amount of starting material. These differences were corrected by the second step: a normalization factor was computed using the mean of acceptable housekeeping gene ratios. Of these housekeeping genes, only those genes for which the relative expression remained unchanged between samples were used.

After normalization, the variance of the normalized set of housekeeping genes (except those affected by the tested condition) was used to generate a confidence interval to test the significance of the gene expression ratios obtained. Ratios outside the 95% confidence interval were determined to be significantly different.

The gene expression ratio was considered as either quantitative or qualitative. Quantitative ratio means that the test and the reference signals were acceptable. Qualitative ratio means that either the test or the reference signal is acceptable. The limits of the quantitative interval were based on the detection curve. For the complete experiment, we set the quantitative range between 3 and 1000 AU in concentration values (arbitrary units, based on the detection curve).

Figure 2 provides a correlation plot between converted Silverquant ratios versus non converted ratios in this gene expression experiment. Ratios are plotted with standard deviations. The scatter plot represents a zoom on the range [-10; 10]. No linear relationship was found between the two sets of data. This experiment clearly shows the influence of the conversion step on the gene expression ratio using a colorimetric detection method.

### *Real time PCR*

The single strand-cDNA (ss-cDNA) was synthesized from 5 μg of total RNA according to the RNA labeling protocol described in the DualChip manual with the following minor modifications: (1) a DNAse treatment of RNA was performed prior to cDNA synthesis; (2) the dNTP mixture contained dGTP, dATP, dTTP and dCTP each at 500μM but no biotinylated dCTP; (3) the second addition of reverse transcriptase was omitted.

Gene specific primers corresponded to the gene sequences present on the microarray. Forward and reverse primers for real-time PCR amplification were designed with the Primer Express Software (PE Applied Biosystems, Foster City, CA, USA).

PCR reaction mixtures contained of 12.5 μl SYBR green PCR Master Mix 2X (PE Applied Biosystems, Foster City, CA, USA), 2.5μl forward primer (3mM), (PE Applied Biosystems, Foster City, CA, USA), 2.5μl reverse primer (3mM) (PE Applied Biosystems, Foster City, CA, USA), 5μl cDNA and 2.5 μl distilled water. PCR reactions without cDNA were performed as template-free negative controls. All PCR reactions were made in duplicates with the following PCR conditions: 2 min at 50 °C, 10 min at 95 °C followed by 40 cycles of 15 s at 95 °C and 1 min at 60 °C in 96-well optical plates (PE Applied Biosystems, Foster City, CA, USA) in the ABI 7000 Sequence Detection System (Perkin-Elmer Life Sciences, Boston, MA, USA). The ABI PRISM 7000 sequence detection system software (version 1.6) was used for data analysis according to the manufacturer's instructions (PE Applied Biosystems, Foster City, CA, USA).

Fluorescence emission was detected for each PCR cycle and the threshold cycle ($C_T$) values were determined. The $C_T$ value was defined as the actual PCR cycle when the fluorescence signal increased above the background threshold. Average $C_T$ values from duplicate PCR reactions were normalized to average $C_T$ values for housekeeping gene from the same cDNA preparations. The ratio of expression of each gene in BCC lines (test samples) vs. cell line pool (reference sample) was calculated as $2^{-(\Delta\Delta CT)}$ of that condition as recommended by Perkin-Elmer where $C_T$ is the threshold cycle and $\Delta\Delta C_T$ is the difference $C_T$ (test gene) - $C_T$ (housekeeping gene) for test sample minus reference sample. Values were reported as an average of triplicate analyses.

The following table 1 compares gene expression ratios obtained by real time PCR and by microarray experiments performed on DualChip human hepato version 1.0 in Silverquant®. Ratios were presented as the mean of 9 experiments for Silverquant detection method and as the mean of 3 experiments for real time PCR. Ratios obtained with Silverquant were either converted or not by the detection curve.

Ratios obtained with Silverquant® and conversion using the detection curve show better correlation with the reference method real-time PCR.

**Table 1**

| Gene | FN | ON | APOJ | FMO5 | CAS7 | PEPT1 | PXR |
|---|---|---|---|---|---|---|---|
| Real Time PCR | 0.14 | 0.7 | 0.12 | 1.88 | 30.25 | 353.52 | 7.92 |
| Microarray Silverquant detection with conversion | 0.09 | 0.82 | 0.05 | 1.37 | 5.42 | 12.89 | 2.1 |
| Microarray Silverquant detection without conversion | 0.48 | 0.92 | 0.52 | 0.92 | 2.94 | 3.75 | 1.54 |

Example 2: quantification of gene expression on arrays with fluorescence labeling and comparison with Silverquant® labeling (and conversion using the detection curve).

The same experiment described in example 1 was conducted, where colorimetric detection was replaced with fluorescence detection. Fluorescence detection, which uses a Cy3-labeled anti-biotin conjugated antibody, was used.

### *Fluorescence detection*

The micro-arrays were incubated for 45 min at room temperature with the Cy3-conjugated IgG Anti biotin (Jackson Immuno Research laboratories, Inc #200-162-096) diluted 1/1000 X Conjugate-Cy3 in the blocking reagent. The micro-arrays were washed 4 times for 2 minutes with Washing buffer and 2 times for 2 minutes with distilled water before being dried.

### *Scanning and quantification*

The hybridized micro-arrays were scanned using a confocal laser scanner ScanArray® 4000XL (PerkinElmer Life Science, USA) at a resolution of 10 μm. To maximize the dynamic range of the assay the same arrays were scanned at different photomultiplier tube (PMT) settings: 50, 70 and 100%. After image acquisition, the scanned 16-bit images were imported to the software, 'ImaGene4.0' (BioDiscovery, Los Angeles, CA, USA), which was used to quantify the signal intensities.

Using the fluorescence Excel template, the fluorescence intensities of each DNA spot (average intensity of each pixel present within the spot) was calculated using local mean background subtraction. A signal was accepted if the average intensity after background subtraction was at least 2 times higher than the local background and higher than the mean of the negative hybridization controls plus 2 times their standard deviation. Very bright element intensities (saturated signals, highly expressed genes) were deemed unsuitable for accurate quantification because they underestimated the intensity ratios. They were excluded from quantitative analysis.

The conversion step has been omitted in this experiment.

The normalization was performed as provided in example 1.

The data analysis workflow is the same than in Silverquant®, except that the limits of the quantitative interval were based on fixed values (not saturated and detected above the background) rather than on the positive detection curve as in Silverquant® detection.

Figure 3 illustrates the fluorescence detection curve obtained in this experiment.

The graph is expressed as signal median (I) as a function of the concentration value in arbitrary units (AU) of the printed detection molecule. The three curves comprise a linear first part and saturated second part. An equation has been calculated in the linear part of each of the three curves: PMT 50% ($y = 8.18\ x - 14831$, $R^2 = 0.9277$), PMT 70% ($y = 25.313\ x - 22726$, $R^2 = 0.9746$), PMT 100% ($y = 55.792\ x - 869.161$, $R^2 = 0.9946$).

*Comparison of ratios in fluorescence (3 PMT settings) and converted Silverquant®*

The gene expression ratios obtained in fluorescence at 3 PMT settings and the Silverquant® converted ratios (experiment of example 1) were plotted with standard deviations. The correction plot is presented in figure 4.

Figure 4 shows a linear relationship between the ratios. The $R^2$ statistic measuring the conformity between ratios for both methods has been computed and corresponds to 0.9869. The scatter plot represents a zoom on the range [-10; 10]. There is a tendency to more inter-experiment variability for greater ratios for both axes. The correlation between the two methods is very good ($R^2 = 0.9869$), showing the interest of converting the Silverquant® detection signal into concentration values. After conversion by the detection curve, Silverquant® detection method gives a gene expression analysis very similar to the data obtained in fluorescence detection.

Example 3: fluorescence detection of interleukin-12 protein on microarray with or without correction by the detection curve.

The experiment was performed using the SignalChip Human Cytokine kit (Eppendorf, Germany) suitable for the detection of 20 cytokines of human origin (IL-1α, IL-1β, IL-1ra, IL-2, IL-4, IL-6, IL-8, IL-10, IL-12p40, IL-12p70, IL-17, TNFα, TNF-RI, TNF-RII, IFNγ, GM-CSF, Eotaxin, MIP-1α, MIP-1β, Rantes).

Each slide contains 8 identical arrays comprising triplicate spots of capture antibodies for each cytokine to be detected (capture probes) and triplicate spots of 13 different concentrations of a control antibody irrelevant to the cytokine field (detection molecule), ranging from 0.025 to 50 μg/ml: 0.025, 0.050, 0.075, 0.10, 0.35, 0.70, 1, 3.5, 7, 10, 15, 20 and 50 μg/ml. These concentrations are equivalent to the following arbitrary units concentrations (AU): 25, 50, 75, 100, 350, 700, 1000, 3500, 7000, 10000, 15000, 20000 and 50000.

A detection curve is constructed from the signals measured from the different concentrations of the detection molecule and is used to convert the signals detected for cytokines possibly present in test samples.

Ten different sample solutions, each containing a predetermined amount of interleukin-12 (IL-12, Biosource, Belgium; 0 to 500 pg) diluted in Dilution Buffer 2 (SignalChip Human Cytokine kit, Eppendorf, Germany), were assayed using the SignalChip Human Cytokine kit. Two slides mounted with 8-well hybridization chambers were blocked for 15 min with Blocking Buffer (SignalChip Human Cytokine kit, Eppendorf, Germany), washed once with Signal Buffer A (SignalChip Human Cytokine kit, Eppendorf, Germany), and individual arrays were contacted with the different solutions. Binding was performed overnight at 22°C under agitation at 1000 rpm in a thermomixer (Eppendorf, Germany), followed by 3 washing steps with Signal Buffer A. A mix of biotinylated primary antibodies (Biotinylated Antibody Solution, SignalChip Human Cytokine kit, Eppendorf, Germany) was contacted with each array for 1 hour at room temperature, followed by 3 washing steps with Signal Buffer A. The Biotinylated Antibody Solution comprised a cocktail of antibodies for recognition of the 20 cytokines of the array and for recognition of control antibody (detection molecule) of the detection curve.

Fluorescence detection was performed using a Cy3-conjugated anti-biotin antibody (Jackson ImmunoResearch, USA; 1000x dilution in Dilution Buffer 2) for 45 min at room temperature, followed by one washing with Signal Buffer A. Hybridization chambers were then removed, and slides were washed once with Signal Buffer A and twice with water. Slides were dried and scanned using ScanArray scanner (Perkin Elmer, USA) at a PMT of 70 and a laser power of 100%. Each array image was quantified using Imagene quantification software (Biodiscovery, USA).

After quantification of the scanned images, raw data intensity for each spot was obtained. Each spot was present in triplicate on the array. After subtraction of the local background, the average of the triplicate values was calculated.

A detection curve was drawn for each array, representing the signal median intensity (I) (averaged and background corrected) of the different spots of the detection molecule as a function of its printed concentration value (AU).

On the basis of this detection curve, the signal (I) of IL-12p40 present in the sample solution contacted with the same array is converted into concentration value (AU). The converted signals for the different IL-12p40 amounts are used to compare data obtained on different arrays and in different experiments.

Figure 5 represents the concentration curve of IL-12p40. The curve is expressed as the signal median (I) as a function of the protein amount of IL-12p40 (pg) contacted with the individual arrays. The first part of the curve, ranging from 0 to 100 pg of IL-12p40 is linear. In the intermediate part of the curve, ranging from 100 to 300 pg, signals are non linear and non saturated. The last part of the curve, ranging from 300 to 500 pg of Il-12 is non linear and saturated.

The concentration curve after conversion of the signal intensity into concentration value is presented in figure 6. IL-12p40 signals from individual arrays are converted using the detection curve generated from the detected signals of the detection molecule (control antibody) printed on the same array. The target concentration curve is expressed as concentration value (AU) (converted) as a function of the amount of IL-12p40 (pg/array). The curve is compared on the same graph to the non-converted signal intensity. Only the non saturated signals are plotted, i.e between 0 and 100 pg IL12p40/array. The non-converted curve is linear in this concentration range and the linear regression is given by the equation: $y = 240.39X$ with $R^2 = 0.9973$.

After conversion, the equation of the curve is: $y = 63.515X$ with $R^2 = 0.9965$. The slope of the curve being known (63.515), it may be used to quantify unknown amounts of IL-12p40 in a sample solution. The steps for quantifying IL-12p40 in a sample solution are the following: the signal median obtained from the binding of IL-12p40 on IL-12 capture probe is converted into a concentration value (AU) based on the detection curve, then the concentration value is divided by the slope of concentration curve (63.515) to obtain the amount of IL-12p40 contacted with the array (pg/array). This value may be further corrected by the dilution factor of the solution contacted with the array compared to obtain the original sample concentration.

Example 4: colorimetric detection of GM-CSF protein on micro-array with or without correction by the detection curve

The same experiment described in example 3 was conducted on 15 different sample solutions containing a predetermined amount of GM-CSF (Biosource, Belgium; 0 to 300 pg), and fluorescence detection was replaced with colorimetric detection. The Silverquant detection kit (Eppendorf, Germany), which uses a gold-labeled anti-biotin conjugated antibody followed by silver precipitation, was used. We strictly followed the user's manual recommendations. Slides were dried and scanned using Silverquant scanner (Eppendorf, Germany). Each image was quantified using Silverquant analysis software (Eppendorf, Germany).

The data analysis software for Signalchip microarrays (Eppendorf, Germany) has been particularly designed to normalize the data, obtain a cytokine profile for each sample, get quantitative information about the cytokine amounts present in the samples, and possibly compare samples processed on different microarrays.

Figure 7 represents the concentration curve of GM-CSF.

The curve is expressed as the signal median (I) as a function of the amount of GM-CSF (pg) contacted with the individual arrays. The first part of the curve, ranging from 0 to 20 pg of GM-CSF is non linear and non saturated. The second part of the curve, ranging from 40 to 300 pg of GM-CSF is non linear and saturated.

Detection curves were generated for each array from the different concentrations of the detection molecule, and they were used to convert the signals from figure 7, thereby converting the signal median intensities into concentration values (AU). The target concentration curve after conversion of the signal intensity into concentration value is presented in figure 8. The target concentration curve is expressed as concentration value (AU) (converted) as a function of the amount of GM-CSF (pg/array). The curve is compared on the same graph to the non-converted signal intensity. Only the non saturated signals are plotted, i.e between 0 and 20 pg GM-CSF/array. The non-converted curve is non linear in this concentration range. However, a linear relationship is obtained for the converted curve and the equation of the curve is: $y = 353.58x$ with $R^2 = 0.9955$. Unexpectedly, quantification is possible in non linear part of the curve as long as the signal is unsaturated. The slope of the curve (353.58) may be used to quantify unknown amount of GM-CSF in a sample solution as disclosed in example 3.

Example 5: Quantification of MIP-1α protein in a sample solution (in fluorescence)

The same experiment as described in example 3 was conducted on several dilutions of MIP-1α (Biosource, Belgium; 0 to 500 pg) and on one dilution of a cytokine mix containing 17 cytokines, among which MIP-1α, at predetermined amounts (Human cytokine 25-plex standard, Biosource, Belgium). The amount of MIP-1α in the mix was 11.5 ng. The vial content of the cytokine 25-plex was resuspended in 500 μl PBS and a 50-fold dilution was performed in Dilution Buffer 2 (SignalChip Human Cytokine kit, Eppendorf, Germany). 87 $\mu$l of this dilution were contacted with one array of a SignaChip Human Cytokine slide. Fluorescence detection was performed as in example 3 with a PMT setting of 50% for the scanning. Signals corresponding to MIP-1$\alpha$ in the samples containing the different dilutions of the single cytokine and in the 25-plex mix were converted as described above with the detection curves constructed from the detection molecule of their respective arrays.

A target concentration curve was constructed, where the concentration value (AU) for the different MIP-1$\alpha$ dilutions were plotted versus the protein amount (pg/array) as provided in figure 9. The equation of the curve in the linear part (from 0 to 80 pg/array) is: $y = 94.962X$ with $R^2 = 0.9991$. The converted concentration value (AU) calculated for MIP-1$\alpha$ present in the Cytokine 25-plex was reported on this detection curve, and the corresponding protein amount was deduced from the curve equation. The calculated protein amount (47 pg), as indicated by a black square on figure 9, corresponds well to the theoretical Biosource value (40 pg). The amount of the target compound in the original sample solution is given by the amount of the target compound in the array incubated solution (47 pg) corrected by a dilution factor of the solution compared to the original sample solution. After correction by the dilution factor, we found 13.5 ng of MIP-1$\alpha$ which is very similar to the predetermined amount in the 25-plex mix (11.5 ng).

Example 6: Cytokine quantification using a calibration curve (in fluorescence)

The same experiment as described in example 4 was conducted, except that the concentrations of the detection curve were as follows: 75AU, 250AU, 1000AU, 2000AU, 3500AU, 7000AU, 10000AU, 15000AU, 20000AU, 30000AU, 40000AU, 50000AU and 60000AU.

Two arrays of a SignaChip Human Cytokine slide (Eppendorf, Germany) were used. One array was contacted with a mix of IL-1$\beta$ and TNF$\alpha$ (10 pg each), and the second array was contacted with a mix of TNF$\alpha$ and IL-4 (20 pg each). The final volume per array was 87 $\mu$l, and the buffer used to dilute the cytokines was Dilution Buffer 2 (SignalChip Human Cytokine kit, Eppendorf, Germany). All 3 cytokines were from Peprotech Inc. (USA). Fluorescence detection was performed as in example 4 with a PMT setting of 50 for the scanning.

Signals corresponding to the cytokines were normalized as described above with the detection curves constructed from the positive detection controls present on their respective arrays. Normalized signals were quantified using concentration curves previously constructed with known amounts of the cytokines (TNF$\alpha$, IL-1$\beta$, IL-4).

Figure 12 shows the detection curves corresponding to each array. Figure 13 shows the concentration curves for each cytokine.

The table below summarizes the results obtained.

**Table 2**

| Sample | Signal (RFU) | Concentration value (AU) | Cytokine amount (pg/array) |
|---|---|---|---|
| 10 pg TNF$\alpha$ | 1216 $\pm$ 73 [1] | 1583 $\pm$ 80 [2a] | 9.4 $\pm$ 0.5 [3] |
| 10 pg IL-1$\beta$ | 6080 $\pm$ 109 [1] | 6802 $\pm$ 107 [2a] | 11.1 $\pm$ 0.3 [3] |
| 20 pg TNF$\alpha$ | 3089 $\pm$ 209 [1] | 3814 $\pm$ 230 [2b] | 22.4 $\pm$ 1.8 [3] |
| 20 pg IL-4 | 11018 $\pm$ 850 [1] | 11983 $\pm$ 647 [2b] | 19.2 $\pm$ 2.8 [3] |
| [1] Fluorescence signal minus local background, averaged for the triplicates. [2a] Calculated using detection curve 1 (figure 12a) [2b] Calculated using detection curve 2 (figure 12b) [3] Calculated using the corresponding concentration curve (figure 13). | | | |

It clearly appears that a same amount of two different cytokines can generate very different values. The signal values for 10 pg of TNF$\alpha$ and IL-1$\beta$ differ by a factor 5, while the signals generated by 20 pg of TNF$\alpha$ and IL-4 differ by a factor >3.5.

These factors are conserved when the fluorescence signals are normalized, but the quantification process using concentration curves completely suppresses this difference.

Cytokines for which the detection process varies significantly, possibly due to the detection efficiency by their respective antibodies, can advantageously be quantified using the present method.

The protein amounts calculated using the normalization/quantification process (table above, last column) correlates well with the experimental values (10 pg and 20 pg).

The quantification of targets using a concentration curve, a described in example 6, is not limited to proteins of the cytokine family, but can be adapted to virtually any biological molecule.

As an example, transcription factors may be quantified using a similar approach, which combines the conversion of signal intensities into concentration values using a detection curve (normalization process), and the subsequent conversion of the concentration values into target amounts using a concentration curve (quantification process).

In the particular case of transcription factors, the capture probes contain a double-stranded DNA part to which the transcription factors specifically bind. The detection molecules, used to build the detection curve, are also made of double-stranded DNA. Detection of the bound targets and of the detection molecules is performed similarly, using primary and secondary antibodies.

The signal intensities of the detection molecules are used to construct the detection curve. The signal intensities of the targets are then normalized using this detection curve and converted into concentration values. Concentration curves can be constructed from known amounts of the targets, and be used to convert the concentration values into target amounts. This can be applied to any target for which a concentration curve can be generated, i.e. any target which is available under a calibrated (quantified) form.

**Claims**

1. A method for a quantification of a target compound (1) (being a polynucleotide or a protein) present in a sample solution, comprising the steps of:

   a) Possibly diluting the target compound (1) by a known dilution factor,
   b) possibly labeling the target compound (1),
   c) putting into contact the target compound (1) with a capture probe (2), in order to allow a specific binding between the said target compound (1) and the said capture probe (2), the said capture probe (2) being fixed upon a surface of a solid support (3) according to an array comprising a density of at least 10 discrete regions per cm$^2$, each of said discrete regions (A) being fixed with a species of capture probe (2) capable of specifically binding to a target compound (1), wherein at least 4 additional discrete regions (B) of the solid support (3) surface are printed with solutions containing known different and increased concentrations of a (labeled) detection molecule (4, 4', 4", 4'''), said detection molecule being from the same family, either a polynucleotide or a protein, as the capture probes (2),wherein each of said discrete regions (A,B) have a diameter comprised between 50 and 500 μm,
   d) detecting by the same detection means signals resulting from the binding between the target compound (1) and its corresponding capture probe (2) and resulting from the printed detection molecule (4, 4', 4", 4''') in the different discrete regions (A, B),
   e) obtaining a detection curve (C) of the detected signals of said detection molecule (4, 4', 4", 4''') in the at least 4 additional discrete regions (B) of the solid support (3) surface as a function of their corresponding printed detection solution concentrations in arbitrary units (AU),
   f) converting the signal obtained from the target compound (1) bound to a specific capture probe (2) into a concentration value presented in arbitrary units (AU) by a conversion step upon the detection curve (C) of the detected signal, and
   g) quantifying the target compound by converting said concentration value into a target amount using a target concentration curve.

2. The method according to claim 1, wherein the quantification of the target compound is performed for signals which are not in the linear part of the detection signal.

3. The method according to claim 1 or 2, wherein the volume of printed solutions is comprised between 0.01 and 5 nl.

4. The method according to any of the claims 1 to 3, wherein the detection molecule is printed in discrete regions with a solid pin.

5. The method according to any of the claims 1 to 3, wherein the detection molecule is deposited on the surface of a solid support in the form of a droplet.

6. The method according to any of the claims 1 to 5, wherein 10 different concentrations of detection molecule solution are printed in different discrete regions.

7. The method according to any of the claims 1 to 6, wherein the concentration of the printed detection molecule solution is comprised between 0.1 and 3000 nM for a detection molecule being a polynucleotide.

8. The method according to claim 8, wherein the concentrations of detection molecule solution are: 0.3, 1, 5, 17.5, 50, 100, 150, 200, 250, and 300 nM.

9. The method according to any of the claims 1 to 8, wherein the concentration of the printed detection molecule solution is comprised between 0.025 and 50 μg/ml for a detection molecule being a protein.

10. The method according to claim 10, wherein the concentrations of detection molecule solution are: 0.025, 0.050, 0.075, 0.10, 0.35, 0.70, 1, 3.5, 7, 10, 15, 20 and 50 μg/ml.

11. The method according to any of the claims 1 to 10, wherein the different concentrations of the printed detection molecule solution spread over at least 2 log concentration.

12. The method according to any of the claims 1 to 10, wherein the different concentrations of the printed detection molecule solution spread over at least 3 log concentration.

13. The method according to any of the claims 1 to 12, wherein the factor separating the different concentrations of the printed detection molecule solution is comprised between 1.2 and 5.

14. The method according to any of the claims 1 to 13, wherein the fixation of capture probes is obtained by linkage of amino groups on activated glass of the solid support bearing aldehyde moiety.

15. The method according to any of the claims 1 to 14, wherein the target concentration curve is generated by contacting different amounts of target compound upon different arrays and converting the signal obtained into concentration value (AU).

16. The method according to claim 15, wherein a coefficient is calculated from the slope of the target concentration curve.

17. The method according to claim 15, wherein a coefficient of the slope of the target concentration curve is obtained by the assay of only one given target amount performed on one array.

18. The method according to claim 16 or 17, wherein the quantification of the target compound in the sample solution is calculated from the coefficient of the slope of the target concentration curve.

19. The method according to claim 15, wherein the quantification of the target compound in the sample solution is calculated from the target concentration curve by converting the target amount into target concentration in the sample solution.

20. The method according to any of the claims 1 to 19, performed on two different samples, wherein the ratio between the concentrations of a target compound in the two sample solutions is obtained by calculating the ratio of the concentration values obtained on the two arrays.

21. The method according to any of the claims 1 to 20, wherein the amount of the target compound in a sample solution is the amount of the target compound in the array incubated solution corrected by the dilution factor of the said solution.

22. The method according to any of the claims 1 to 21, wherein the signals obtained on the different discrete regions are fluorescent.

23. The method according to any of the claims 1 to 21, wherein the signals obtained on the different discrete regions are precipitate.

24. The method according to claim 23, wherein the precipitate is a metallic precipitate.

25. The method according to claim 24, wherein the metallic precipitate is obtained by chemical reduction of silver in the presence of colloidal gold particles coupled to the bound target compound.

26. The method according to claim 24, wherein the metallic precipitate is obtained in the presence of an enzyme.

27. The method according to any of the claims 1 to 26, wherein the target compound is a cytokine protein selected from the group consisting of: IL-1α, IL-1β, IL-1ra, IL-2, IL-4, IL-6, IL-8, IL-10, IL-12 p40, IL-12 p70, IL-17, TNFα, TNF-RI, TNF-RII, IFNγ, GM-CSF, Eotaxin, MIP-1α, MIP-1β, Rantes.

**28.** The method according to any of the claims 1 to 27, for simultaneous quantification of at least two proteins differing in signal by a factor of at least 2 when present at the same amount.

**29.** A program executable on a programmable device containing instructions, which when executed performing at least the steps e) to g) and possibly to step d) to g) of the method according to any of the preceding claims 1 to 28.

**30.** A diagnostic and/or quantification system or kit or quantification of a target compound (1) being a polynucleotide or a protein and being present in a solution, which comprises:

- a solid support (3) with an array surface comprising at least 10 discrete regions per cm$^2$, each of said discrete regions (A) being fixed with one species of a capture probe (2) capable of specifically binding to a target compound (1), wherein at least 4 additional discrete regions (B) are printed with solutions containing known different and increased concentrations of a detection molecule (4, 4', 4", 4'''), said detection molecule (4, 4', 4", 4''') being from the same family, either a polynucleotide or a protein, as the capture probes, wherein each of said discrete regions (A,B) have a diameter comprised between 50 and 500 μm,
- the program according to the claim 29 executable on a programmable device containing instructions when executed, performed the following steps :

d) possibly detecting by the same detection means signal resulting from the binding between target compound (1) and its corresponding capture probes (2) and resulting from the printed detection molecule (4, 4', 4", 4''') in the different discrete regions (A, B),
e) obtaining a detection curve of the detected signal of the detection molecule (4, 4', 4", 4''')in the at least 4 additional discrete regions (B) of the solid support (3) surface as a function of their corresponding printed detection solution concentrations in arbitrary units (AU),
f) converting the signal obtained from the target compound (1) bound to a specific capture probe (2) into a concentration value presents in arbitrary units (AU) by a conversion step upon the detection curve (C) of the detected signal and,
g) quantifying the target compound by converting said concentration value into a target amount using a target concentration curve.

FIG. 1

FIG. 2

**Fluorescence detection curve**

FIG. 3

FIG. 4

FIG. 5

FIG. 6

FIG. 7

FIG. 8

FIG. 9

FIG. 10

FIG. 11

a.

Detection curve 1 (array 1)

b.

Detection curve 2 (array 2)

FIG. 12

FIG. 13

European Patent
Office

## EUROPEAN SEARCH REPORT

Application Number

EP 07 11 8308

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/154299 A1 (HARVEY MICHAEL A [US] ET AL) 13 July 2006 (2006-07-13) <br> * page 1, paragraphs 2,3,9,15-17 * <br> * page 2, paragraphs 19-22,31 * <br> * page 3, paragraph 33-39 * <br> * page 4 - page 5; claims 1,13,14,19,20,25,27,28 * <br> ----- | 1-30 | INV. <br> G01N33/543 <br> C12Q1/68 <br> G01N33/68 |
| X | EPPENDORF AG: "DualChip microarrays" INSTRUCTION MANUAL , VERSION 2.0, [Online] 24 November 2005 (2005-11-24), XP002415465 Retrieved from the Internet: URL:http://www.eppendorfna.com/manuals/man uals-BS.asp> <br> * page 8 - page 11 * <br> * page 48 - page 52 * <br> * figure 18 * <br> ----- | 1-30 | |
| X | EPPENDORF AG: "DualChip evaluation software" USER MANUAL VERSION 1.1, [Online] 24 November 2005 (2005-11-24), XP002415466 Retrieved from the Internet: URL:http://www.eppendorfna.com/manuals/man uals-BS.asp> <br> * page 7 - page 9; figures 1,2 * <br> * page 13 - page 14 * <br> ----- | 1-30 | TECHNICAL FIELDS SEARCHED (IPC) <br><br> G01N <br> C12Q |
| A | US 2006/115840 A1 (BOUCHER SHERRI [CA] ET AL) 1 June 2006 (2006-06-01) <br> * page 1, paragraphs 13,14 * <br> * page 2, paragraphs 15,18-35 * <br> * figure 1 * <br> ----- | 1-30 | |

The present search report has been drawn up for all claims

| Place of search | Date of completion of the search | Examiner |
|---|---|---|
| Munich | 13 February 2008 | Pilch, Bartosz |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ..............................................................................

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 07 11 8308

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2008

| Patent document cited in search report | Publication date | Patent family member(s) | Publication date |
|---|---|---|---|
| US 2006154299 A1 | 13-07-2006 | NONE | |
| US 2006115840 A1 | 01-06-2006 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9511995 A
- WO 0072018 A
- US 5270167 A
- US 4731325 A
- EP 0301141 A
- EP 0393868 A
- WO 8602733 A
- EP 0063810 A
- US 5821060 A
- WO 9504160 A
- WO 2004064482 A
- US 20060115840 A
- WO 2006027088 A
- WO 00072018 A1
- US 5561071 A

- GB 3319838 A
- WO 9729212 A
- US 5632957 A
- US 3817837 A
- US 3850752 A
- US 3939350 A
- US 3996345 A
- US 4277437 A
- US 4275149 A
- US 4366241 A
- US 5605662 A
- US 5552270 A
- WO 9828444 A
- WO 0218288 A
- WO O185978 A

**Non-patent literature cited in the description**

- **DE LONGUEVILLE et al.** *Biochemical Pharmacology,* 2002, vol. 64, 137-149
- **CHEN et al.** *J. Biomed. Optics,* 1997, vol. 2, 364-74
- **SCHENA et al.** *Proc. Natl. Acad. Sci. USA,* 1996, vol. 93, 10614-10619
- **KRENSKY et al.** *Nucleic Acids Research,* 1987, vol. 15, 2891-2909

- **JOOS et al.** *Anal. Biochem.,* 1997, vol. 247, 96-101
- **CHRISEY et al.** *Nucleic Acids Research,* 1996, vol. 24, 3031-3039
- **ECKERT et al.** *Biomaterials,* 2000, vol. 21, 441